# EUROPEAN PATENT APPLICATION

(11) **EP 2 887 244 A1**
(43) Date of publication of application: **24.06.2015**
(21) Application number: 14198253.8
(22) Date of filing: 16.12.2014
(51) Int. Cl.: G06F 19/00, G06Q 50/22

(54) **Risk management approach to oversight of institutional review boards**

(30) Priority: 19.12.2013 US 201314135016
(71) Applicant: Medtronic Inc., Minneapolis, Minnesota 55432 (US)
(72) Inventor: OLESON, Kimberly A., Shoreview, MN Minnesota 55126 (US); FAY, Margaret F., Suwanee, GA Georgia 30024 (US)
(74) Representative: Hughes, Andrea Michelle

(57) **Abstract**

A computer system receives, before a start of a trial of a medical therapy, data that configure the computer system to detect alert conditions indicative of occurrences of failure modes identified as being potentially associated with an institutional ethics committee such as an Institutional Review Board (IRB) that reviews the trial. Respective mitigation procedures are defined for the failure modes before the start of the trial. The mitigation procedures indicate procedures to mitigate occurrences of the failure modes. Furthermore, the computer system receives IRB activity data indicative of activities performed by the IRB. The computer system detects, based on the IRB activity data that one of the alert conditions has occurred. The alert condition is indicative of an occurrence of a given one of the failure modes. In response to detecting that the alert condition has occurred, the computer system outputs an alert.

## Description

### BACKGROUND

In general, sponsoring organizations (referred to herein as "sponsors") develop medical therapies with the intent of bringing the medical therapies to market. For instance, a sponsor may develop a new medical device, a new drug, a new biological compound, a new use for an existing medical device or drug, a new medical treatment process or procedure, or another type of medical therapy. In some instances, a medical therapy may be referred to as an investigational product. A study treatment may be a drug, device, therapy or process under investigation in a clinical trial which has an effect on outcome of interest in a study: e.g., health-related quality of life, efficacy, safety, and pharmacoeconomics. A study treatment may also be referred to as an intervention, a therapeutic intervention, a medical product, or an investigational product.

Before a sponsor can bring a medical therapy to market, the medical therapy must undergo various trials to ensure the safety and efficacy of the medical therapy. For instance, the medical therapy may undergo a clinical trial in which the medical therapy is used on actual persons (also referred to as human subjects). If the trials demonstrate that the medical therapy is safe and effective, the medical therapy may be approved for market.

To conduct a trial on a medical device, a sponsor may first develop a trial protocol. The trial protocol describes how to conduct the trial. For instance, the trial protocol may provide instructions on how to recruit study subjects, how to collect information from the patients, how to administer the medical therapy, what information to collect from the patients, and how to perform other aspects of the trial.

The sponsor may then engage (e.g., hire) one or more research organizations (i.e., investigators) to conduct the trial on the medical therapy. To help ensure that the results of the trial are not biased, the investigators may be organizations that are independent of the sponsor. The sponsor instructs the investigators to conduct the trials in accordance with the applicable global regulatory requirements and the trial protocol developed for the medical therapy. The investigators may provide data associated with the trial to the sponsor during the trial and/or upon completion of the trial.

Furthermore, prior to initiating the trial, an investigator engaged to conduct the trial may submit the trial protocol and other information about the trial to an institutional review board (IRB) or institutional ethics committee (IEC). The IRB reviews the trial protocol and other information about the trial to determine whether the trial conforms to legal and ethical guidelines (e.g., governmental regulations). If the trial conforms to such legal and ethical guidelines, the IRB may indicate to the sponsor that it is acceptable to proceed with the trial. The sponsor may then instruct the investigator to begin the trial. On the other hand, if the trial does not conform to such legal and ethical guidelines, the IRB may indicate flaws in the trial to the sponsor. Furthermore, after the trial begins, the IRB may conduct continuing reviews of the trial to ensure that the trial continues to conform to the legal and ethical guidelines. For instance, in the United States of America (U.S.A.), the IRB may be responsible for compliance review and oversight of clinical research with respect to 21 C.F.R. parts 50 and 56. Furthermore, in the U.S.A., the IRB may provide reports regarding the trial to the United States Food and Drug Administration (F.D.A.).

In general, the IRB does not interact directly with the sponsor when conducting initial or continuing reviews of the trial. However, the IRB may interact with the investigator when conducting initial and continuing reviews of the trial. Thus, the investigator may act as an interface between the sponsor and the IRB. Limiting interaction between the sponsor and the IRB may help to prevent the sponsor from biasing the decisions of the IRB. Because the interactions between the sponsor and the IRB are limited, the investigator may be responsible for assuring the sponsor that the IRB itself complies with regulations governing the conduct of IRBs. In other words, the sponsor relies on the investigator to interface with the IRB.

At the end of the trial, the sponsor may submit an application to a regulatory agency, such as the United States F.D.A. The application may include the data provided by the investigators. The regulatory agency may then decide, on the basis of the data in the application and reports submitted by the IRB, whether to allow the sponsor to bring the medical therapy to market.

For various reasons, the IRBs may not perform their roles correctly. Failure of an IRB to correctly perform its role may jeopardize or delay the approval of the medical therapy by the United States F.D.A. For example, certain failures of the IRB may result in fines or other penalties against the sponsor. In another example, the United States F.D.A. may require particular actions to correct particular failures of the IRB, resulting in delays. Such delays may be particularly costly in the context of a patented medical therapy where each day of delay prior to the medical therapy's approval for sale may have a significant impact on the amount of revenue the sponsor can earn from selling the medical therapy.

### SUMMARY

This disclosure describes techniques performed by one or more computer systems to facilitate risk-based review of actions of Institutional Review Boards (IRBs) prior to and during conduct of trials of medical therapies. That is, the techniques of this disclosure may provide a process, based on quality risk management principles and tools, that addresses particular gaps in the risk oversight scheme for trials of medical therapies. In accordance with some techniques of this disclosure, a computer system may receive configuration data before a trial starts. The configuration data may configure the computer system to detect alert conditions indicative of occurrences of risks identified as being potentially associated with an IRB that reviews the trial. In addition, the computer system may receive IRB activity data indicative of activities performed by the IRB. In some examples, the IRB activity data may be generated by the IRB as part of the IRB conducting initial and continuing reviews of the trial. The computer system may detect, based on the IRB activity data, one of the alert conditions has occurred. In response to detecting that the alert condition has occurred, the computer system may output an alert indicative of an occurrence of a particular one of the failure modes. The alert may be associated with a mitigation procedure defined for the particular failure mode before the start of the trial.

In one example, this disclosure describes a method comprising: receiving, by a computer system, before a start of a trial of a medical therapy, data that configure the computer system to detect an alert condition indicative of an occurrence of a failure mode identified as associated with an Institutional Review Board (IRB) review of the trial, wherein a mitigation procedure is defined for the failure mode before the start of the trial, the mitigation procedure indicating a procedure to mitigate the occurrence of the failure mode; receiving, by the computer system, IRB activity data indicative of activities performed by the IRB; detecting, by the computer system based on the IRB activity data, that the alert condition has occurred; and in response to detecting the alert condition has occurred, outputting, by the computer system, an alert.

In another example, this disclosure also describes a computer system that comprises one or more processing units that are configured to: receive, before a start of a trial of a medical therapy, data that configures the computer system to detect an alert condition indicative of an occurrence of a failure mode identified as associated with an Institutional Review Board (IRB) review of the trial, wherein a mitigation procedure is defined for the failure mode before the start of the trial, the mitigation procedure indicating a procedure to mitigate the occurrence of the failure mode; receive IRB activity data indicative of activities performed by the IRB; detect, based on the IRB activity data, that the alert condition has occurred; and in response to detecting the alert condition has occurred, output an alert.

In another example, this disclosure also describes a non-transitory computer-readable storage medium that stores computer-executable instructions that, when executed, configure a computer system to: receive, before a start of a trial of a medical therapy, data that configures the computer system to detect an alert condition indicative of an occurrence of a failure mode identified as associated with an Institutional Review Board (IRB) review of the trial, wherein a mitigation procedure is defined for the failure mode before the start of the trial, the mitigation procedure indicating a procedure to mitigate the occurrence of the failure mode; receive IRB activity data indicative of activities performed by the IRB; detect, based on the IRB activity data, that the alert condition has occurred; and in response to detecting that the alert condition has occurred, output an alert.

The details of one or more examples of the techniques are set forth in the accompanying drawings and the description below. Other features, objects, and advantages of the techniques will be apparent from the description, drawings, and from the claims.

### BRIEF DESCRIPTION OF DRAWINGS

FIG. 1 is a conceptual diagram illustrating an example environment in which the techniques of this disclosure may be implemented.
FIG. 2 is a conceptual diagram illustrating an example Institutional Review Board (IRB) process, in accordance with one or more techniques of this disclosure.
FIG. 3 is a flowchart illustrating an example operation performed by a sponsor of a trial of a medical therapy prior to the start of a trial of a medical therapy, in accordance with one or more techniques of this disclosure.
FIG. 4 is a flowchart illustrating an example operation performed in part by a computer system after the trial starts, in accordance with one or more techniques of this disclosure.
FIG. 5 is a flowchart illustrating an example operation performed by a computer system, in accordance with one or more techniques of this disclosure.
FIG. 6 is a conceptual diagram illustrating an example table for identifying failure modes.
FIG. 7 is a conceptual block diagram illustrating an example configuration of a computer system.

### DETAILED DESCRIPTION

In general, sponsors develop medical therapies with the intent of bringing the medical therapies to market. Before a sponsor can bring a medical therapy to market, the medical therapy must undergo various trials to ensure the safety and efficacy of the medical therapy. To conduct a trial on a medical device, a sponsor may first develop a trial protocol that describes how to conduct the trial. The sponsor may then engage (e.g., hire) an investigator to conduct the trial on the medical therapy. Prior to initiating the trial, the investigator may submit the trial protocol and other information about the trial to an institutional review board (IRB). Under United States F.D.A. regulations, an IRB is an appropriately constituted group that has been formally designated to review and monitor biomedical research involving human subjects. In other countries, a similarly constituted group may be known as an Ethical Review Board, an Institutional Ethics Committee (IEC), etc.

The IRB reviews the trial protocol and other information about the trial to determine whether the trial conforms to legal and ethical guidelines (e.g., governmental regulations). As part of an initial review of the trial, the IRB may determine whether a trial protocol for the trial complies with applicable guidelines (e.g., regulations) and local IRB standards. Furthermore, as part of the initial review of the trial, the IRB may determine whether case report forms (CRFs) for the trial comply with applicable guidelines. A CRF may be a printed, optical, or electronic document designed to record all required information to be reported to the sponsor for each study subject. In addition, as part of the initial review of the trial, the IRB may determine whether patient consent forms comply with applicable guidelines.

For various reasons, the IRB may not perform its role correctly in compliance with applicable regulations or local IRB standards. In other words, IRB failures may occur. Failure of an IRB to correctly perform its role may jeopardize or delay approval of the medical therapy. For example, the IRB may fail to keep adequate meeting minutes. In another example, the IRB may have inadequate written procedures or may fail to follow written procedures. In another example, the IRB may fail to have the majority of members present during convened meetings. In another example, the IRB may inappropriately use expedited review. In another example, the IRB may fail to conduct continuing review of a trial. In another example, the IRB may fail to have a non-scientific member present during IRB meetings. In another example, the IRB may fail to maintain IRB member rosters. In another example, the IRB may fail to appropriately make significant risk/non-significant risk determinations. In another example, the IRB may fail to make prompt reports to the United States F.D.A.

In general, IRB failures are not detected until after the failure has occurred or after the conclusion of the trial. For instance, IRB failures may typically be detected during a final governmental review of the trial. Detection of an IRB failure at the conclusion of the trial may result in serious study integrity or financial repercussions to the sponsor. Thus, even if one or more computer systems stored data needed to detect IRB failures, such computer systems do not organize or utilize this data in a manner that facilitates use of such data to determine that IRB failures have occurred.

Detection of IRB failures early in the trial or prior to initiation of the trial may result in preservation of study integrity or significant cost savings to the sponsor. In general, this disclosure describes techniques performed by one or more computer systems to facilitate risk-based review of the conduct of IRBs that review trials of medical therapies. That is, the techniques of this disclosure enable computer systems to organize and utilize data in a manner that facilitates the use of such data to determine that IRB failures have occurred. Such risk-based review of the conduct of IRBs may enable early detection of IRB failures. As described in this disclosure, a sponsor may develop a medical therapy. In addition, the sponsor may plan a trial to test whether the medical therapy is safe and effective. As part of planning the trial, the sponsor may identify failure modes associated with activities of an IRB tasked with reviewing the trial for compliance with legal and ethical guidelines (e.g., regulations). Such failure modes may also be referred to as "risks." The sponsor may also identify alert conditions associated with the failure modes and may define mitigation procedures to be performed when the alert conditions occur. Such mitigation procedures may also be referred to as "action plans."

In addition to identifying the failure modes and alert conditions associated with the failure modes, the sponsor may assign priority classifications to the failure modes. The priority classification assigned to a failure mode may be based on how an occurrence of the failure mode may affect the desired endpoints or integrity of the trial. The sponsor may define the mitigation procedures based on the priority classifications assigned to the risks.

Subsequently, the sponsor may engage (e.g., hire) one or more investigators to conduct a trial of a medical therapy developed by the sponsor. In addition, each of the one or more investigators may engage an IRB to provide initial and continuing review of the trial for which the F.D.A. has granted approval to proceed. After the IRB reviews and approves the trial, the sponsor may start the trial. When the sponsor starts the trial, the sponsor may instruct the one or more investigators to start the trial. In accordance with the techniques of this disclosure, the IRB may provide IRB activity data to the computer system. The IRB activity data may include data regarding the IRB and the IRB's initial and continuing review of the trial. For instance, the IRB activity data may include one or more of: data indicating meeting minutes of the IRB, credentials of the IRB, and required reports to the sponsor that are generated by the IRB. Before the trial begins, the sponsor or other entity may configure the computer system to detect, based on the IRB activity data, the occurrence of the alert conditions. Computer system reconfiguration may also occur after the start of the trial and prior to an end of the trial.

When the computer system determines that one of the alert conditions has occurred, the computer system may output an alert to a mitigation team. In some instances, the computer system may generate the alert prior to the start of the trial. Furthermore, in some instances, the computer system may generate the alert after the start of the trial and prior to an end of the trial. In some instances, the mitigation team includes one or more of the sponsors, monitors, contract research organizations (CROs), and/or data management, safety, quality, compliance or regulatory staff. The alert may be associated with one of the previously-defined mitigation procedures. The mitigation team may perform the previously-defined mitigation procedure in response to receiving the alert. For instance, the mitigation team may interact with the IRB in response to receiving the alert. In this way, conduct of the trial may be based on the failure modes identified by the sponsor.

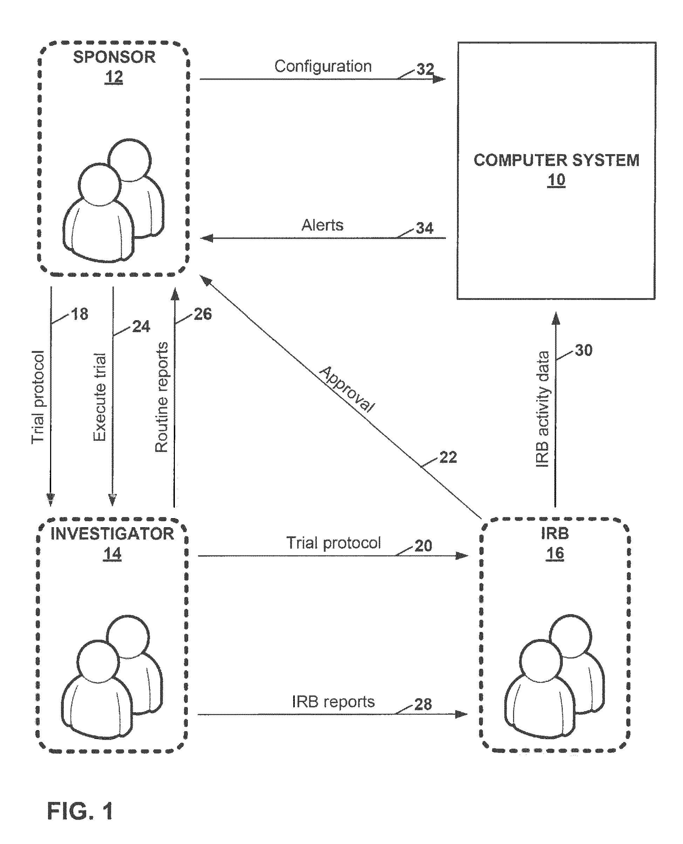

FIG. 1 is a conceptual diagram illustrating an example environment in which the techniques of this disclosure may be implemented. In the example of FIG. 1, the environment includes a computer system 10, a sponsor 12, an investigator 14, and an IRB 16. In other examples, the environment may include more, fewer, or different organizations and systems. In the example of FIG. 1, sponsor 12, investigator 14, and IRB 16 are shown to include multiple persons to indicate that one or more persons may be associated with each of sponsor 12, investigator 14, and IRB 16.

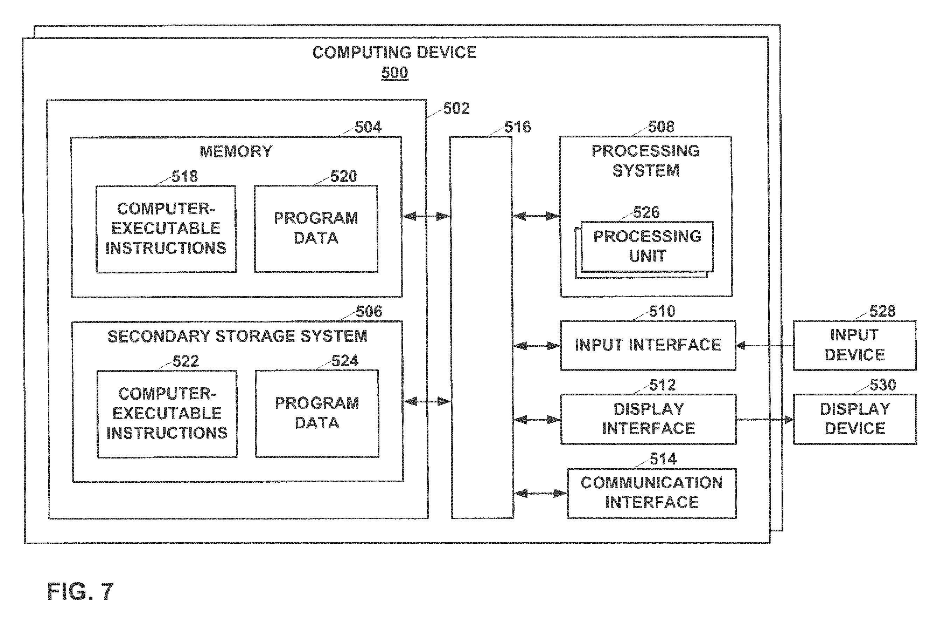

Computer system 10 includes one or more computing devices. For example, computer system 10 may include one or more server computers, personal computers, mainframe computers, storage devices, network devices, or other types of physical devices that process data. FIG. 7, described below, is a block diagram illustrating example components of computer system 10. In some examples where computer system 10 includes more than one computing device, the computing devices of computer system 10 may be geographically dispersed.

In some examples, computer system 10 may appear, from the perspective of sponsor 12, investigator 14, and/or IRB 16, to exist in "the cloud," i.e., as one or more devices interconnected and accessible via a network, such as the Internet. In other examples, computing devices of computer system 10 may be physically located at one or more sites associated with sponsor 12, investigator 14, and/or IRB 16.

Sponsor 12 develops a medical therapy. For example, sponsor 12 may develop a medical device, a drug, a biological compound, surgical procedure, or a new indication for an existing medical device, drug, or compound. Before sponsor 12 is allowed to bring the medical therapy to market, a regulatory agency may require sponsor 12 to prove that the medical therapy is safe and effective.

In order to prove to the regulatory agency that the medical therapy is safe and effective, sponsor 12 may sponsor a trial of the medical therapy. Sponsor 12 may sponsor various types of trials of the medical therapy. For example, sponsor 12 may sponsor a clinical trial, a non-clinical trial, a bench trial, or another type of investigation into whether the medical therapy is safe and effective. In some instances, a trial may be referred to as an investigation, a test, or a study.

In some examples, sponsor 12 may delegate the tasks of designing and conducting the trial to a third-party organization, such as a contract research organization (CRO) or a physician-initiated trial. However, for ease of explanation, the remainder of this disclosure assumes that a sponsor of a trial designs and conducts the trial.

Certain problems may arise if sponsor 12 were to conduct the trial by itself. For instance, sponsor 12 may have a financial interest in proving that the medical therapy is safe and effective. For this reason, sponsor 12 may deliberately or unconsciously distort or bias the results of the trial in order to show that the medical therapy is safe and effective.

To avoid the problems that may arise if sponsor 12 were to conduct the trial, sponsor 12 may engage (e.g., hire, contract with, etc.) investigator 14 to conduct the trial on behalf of sponsor 12. Investigator 14 may be an organization that is independent of sponsor 12. In some instances, investigator 14 may be a CRO.

Before investigator 14 starts work on the trial, sponsor 12 may develop a trial protocol. After developing the trial protocol, sponsor 12 may provide the trial protocol to investigator 14. In the example of FIG. 1, arrow 18 indicates sponsor 12 providing the trial protocol to investigator 14.

The trial protocol may include one or more documents that describe the objectives, design, methodology, statistical considerations, and organization of the trial. For example, the trial protocol may indicate what types of study subjects investigator 14 is to recruit, how to obtain consent from study subjects, what types of required testing to perform, what types of data to collect from study subjects, the length of the trial, and other details regarding the trial. Study data may be prospectively collected or retrospectively ascertained from existing electronic repositories of data (e.g., electronic health records). The study subjects are persons who participate in the trial. Some of the study subjects may receive the medical therapy. Other study subjects are "controls," meaning that such study subjects do not actually receive the investigational medical therapy. In some instances, study subjects may also be referred to as the "subjects" or "participants" of the trial.

The trial protocol may include one or more case report forms (CRFs), trial questionnaires, subject diaries or similar data collection tools. Each of the CRFs may be a paper or electronic questionnaire or similar tool designed to collect trial data (i.e., data generated by conducting the trial). As investigator 14 conducts the trial, investigator 14 may enter trial data into the CRFs. A CRF (or sometimes a case record form) may be a printed, optical, or electronic document designed to record all required information to be reported to the sponsor for each study subject. In some instances, one or more items of an electronic CRF maybe pre-populated (e.g., by computer system 10). Pre-populated items of an electronic CRF may be items that are part of the electronic CRF (or data collection device) that are not enterable/modifiable. These data may be stored in a study database. For example, a required item of an electronic CRF, such as "years" for an age question, may be pre-populated into the electronic CRF based on other data.

Investigator 14 may submit the trial protocol and other information about the trial to IRB 16. In the example of FIG. 1, arrow 20 indicates investigator 14 providing the trial protocol and other information to IRB 16. IRB 16 reviews the trial protocol and other information about the trial to determine whether the trial conforms to legal and ethical guidelines (e.g., governmental regulations). If the trial conforms to such legal and ethical guidelines, IRB 16 may indicate to sponsor 12 that it is acceptable to proceed with the trial. In the example of FIG. 1, arrow 22 indicates the approval provided by IRB 16 to sponsor 12. Sponsor 12 may then instruct investigator 14 to begin the trial. Arrow 24 indicates the instructions from sponsor 12 to investigator 14 to begin the trial. On the other hand, if the trial does not conform to such legal and ethical guidelines, IRB 16 may indicate flaws in the trial to sponsor 12.

After the trial begins, investigator 14 may provide routine reports on the progress of the trial to sponsor 12. Arrow 26 in FIG. 1 indicates the routine reports provided by investigator 14 to sponsor 12. In addition, during execution of the trial, investigator 14 may submit IRB reports to IRB 16. The IRB reports may include reports regarding the progress of the trial, reports of deaths, adverse events, and/or severe adverse events, and so on. Arrow 28 in FIG. 1 indicates the IRB reports provided from investigator 14 to IRB 16. IRB 16 may conduct continuing reviews of the trial to ensure that the trial continues to conform to the legal and ethical guidelines.

In accordance with the techniques of this disclosure, IRB 16 may provide IRB activity data to sponsor 12. Activity data can be entered by sponsor 12 or provided by IRB 16 to computer system 10. Arrow 30 indicates IRB 16 providing the IRB activity data to computer system 10. The IRB activity data may include various types of information about IRB 16. For example, the IRB activity data may include data that indicate the activities of IRB 16. For instance, the IRB activity data may include meeting minutes of IRB 16, reports generated by IRB, and so on. In another example, the IRB activity data may include information about IRB 16. For instance, the IRB activity data may include credentials of IRB 16, credentials and curriculum vitae of members of IRB 16, and so on.

IRB 16 may provide the IRB activity data to computer system 10 at various points during the lifecycle of the trial. For example, IRB 16 may provide IRB activity data to computer system 10 after IRB 16 has completed its initial review of the trial protocol. In another example, IRB 16 may provide IRB activity data to computer system 10 after IRB 16 reviews IRB reports submitted to IRB 16 by investigator 14 during execution of the trial. In another example, IRB 16 may provide IRB activity data to computer system 10 at the conclusion of the trial.

IRB 16 may provide the IRB activity data to computer system 10 in different ways. For example, computer system 10 may provide a user interface (e.g., a web interface, a graphical user interface, etc.) that enables IRB 16 (or users associated with IRB 16) to input the IRB activity data. In another example, IRB 16 may provide paper or electronic copies of documents to sponsor 12, investigator 14, or another party that enters the IRB activity data. Furthermore, in some examples, computer system 10 parses the content of documents containing IRB activity data to extract and store particular data items from the documents.

Furthermore, in accordance with the techniques of this disclosure, sponsor 12 may implement a risk-based monitoring regime with regard to risks associated with IRB 16. Risk-based monitoring is an approach to monitoring that may use risk designation to determine responses to occurrences of alert conditions associated with identified risks. A goal of risk-based monitoring is to increase an overall reliability of the trial data and to reassess the risk factors on a regular basis in order to ensure the overall quality and integrity of the trial data and subject safety. In some instances, risk-based monitoring may enable sponsor 12 to identify trends and data reporting errors in a way that is not possible by reviewing the work of IRB 16 at the end of a trial. The potential benefits of risk-based monitoring may include the ability to identify trends, anomalies, improved productivity using electronic data collection systems to identify missing or incomplete data, real-time response to system-generated alerts, and so on.

In another example, risk-based monitoring may enable sponsor 12 to use real time alerts, notifications, flags and escalation alerts for triggered responses. Risk-based monitoring may also minimize lags in data entry or redundant data entry using integrated systems and tools. Risk-based monitoring may also enable sponsor 12 to eliminate duplicate tasks and detect systematic data entry patterns or problems. Furthermore, risk-based monitoring may enable sponsor 12 to conduct performance analysis to predict future investigation site or investigator selection and patient accrual rates based upon past performance analysis. In another example, risk-based monitoring may enable sponsor 12 to improve quality through integration of predictive analytics and real time data analysis. In addition, risk-based monitoring may enable proactive decision-making through the use of escalations, and eliminate overlap in duplicative tasks.

When sponsor 12 implements a risk-based monitoring regime, sponsor 12 may identify the failure modes (i.e., risks) associated with IRB 16. In addition, sponsor 12 describe each of the failure modes, describe the severity (i.e. criticality) of each of the failure modes, describe detection plans for each of the failure modes, describe safeguards against the occurrence of each of the failure modes, describe mitigation plans for each of the failure modes, and other information regarding the failure modes.

There may be a variety of failure modes (i.e., risks) associated with IRB 16. For example, the failure modes associated with IRB 16 may include problems associated with informed consent forms (ICFs). In this example, sponsor 12 may inadvertently generate ICFs that have missing or incorrect elements. Furthermore, in this example, IRB 16 may be responsible for detecting that the ICFs have missing or incorrect elements. However, IRB 16 may not detect that the ICFs have missing or incorrect elements. Accordingly, sponsor 12 may identify, as a failure mode associated with IRB 16, that IRB 16 approves ICFs that have missing and/or wrong required elements. Thus, the failure modes identified as being potentially associated with IRB 16 may include a failure by IRB 16 to ensure that ICFs used in the trial include all required elements.

In a similar example, different jurisdictions have different requirements (i.e., local requirements) for ICFs. In this example, IRB 16 may be responsible for verifying that the ICFs generated by sponsor 12 conform to the local requirements. Accordingly, in this example, sponsor 12 may identify, as a failure mode associated with IRB 16, that IRB 16 approves ICFs that do not conform to the applicable local requirements. Thus, the failure modes identified as being potentially associated with IRB 16 may include a failure by IRB 16 to ensure that ICFs conform to local requirements.

In another example, sponsor 12 may identify, as a failure mode associated with IRB 16, that IRB 16 approves ICFs without ensuring that necessary translations of the ICFs have been certified. In another example, sponsor 12 may identify, as a failure more associated with IRB 16, that IRB 16 does not review ICFs within an appropriate time frame. In another example, sponsor 12 may identify, as a failure mode associated with IRB 16, that IRB 16 fails to ensure that investigator 14 uses correct ICF translations at particular investigation sites. An investigation site may be a location or facility at which an investigator conducts at least a portion of a trial of a medical therapy. In another example, sponsor 12 may identify, as a failure mode associated with IRB 16, that IRB 16 fails to ensure that investigator 14 has translators at investigation sites when obtaining informed consent from participants.

In another example, IRB 16 may be responsible for determining rates at which investigation sites deviate from regulations or the protocol regarding ICFs. In this example, IRB 16 may fail to take action with respect to investigation sites having unacceptable protocol deviation rates for ICFs (e.g., site investigator fails to obtain written consent prior to study procedures). Accordingly, in this example, sponsor 12 may identify, as a failure mode associated with IRB 16 that IRB 16 fails to take action with respect to investigation sites having unacceptable protocol deviation rates for ICFs.

In another example, sponsor 12 requires site investigators to complete one or more financial disclosure documents as part of preparing the trial. In this example, the one or more financial disclosure documents identify potential investigator conflicts of interest which can affect the reliability of data that is submitted to regulatory agencies. IRB 16 may be responsible for verifying that all potential conflicts of interest have been appropriately disclosed via the one or more financial disclosure documents. However, IRB 16 may approve the trial despite inadequate financial disclosure documents. Accordingly, sponsor 12 may identify, as a failure mode associated with IRB 16, that IRB 16 approves the trial when the one or more financial disclosure documents are inadequate.

In another example, investigator 14 and IRB 16 may have conflicts of interest that may impact the trial. Such conflicts of interest may jeopardize the objectivity of the trial. In general, IRB 16 is responsible for verifying that investigator 14 and IRB 16 do not have such conflicts of interest. Accordingly, sponsor 12 may identify, as a failure mode associated with IRB 16, that IRB 16 approves the trial when investigator 14 and/or IRB 16 have conflicts of interest that may jeopardize the objectivity of the trial. Thus, the failure modes identified as being potentially associated with IRB 16 include approval of the trial by IRB 16 despite a conflict of interest by IRB 16 or an investigator that performs the trial.

Other failure modes associated with IRB 16 may involve the trial protocol. For example, sponsor 12 may identify, as a failure mode associated with IRB 16, that IRB 16 approves the trial when there are missing and/or wrong elements in the original trial protocol. Thus, the failure modes identified as being potentially associated with IRB 16 may include approval of the trial by IRB 16 despite missing or incorrect elements in a trial protocol for the trial. In another example, sponsor 12 may identify, as a failure mode associated with IRB 16, that a cycle review time of the trial protocol by IRB 16 does not satisfy the applicable parameters.

In another example, IRB 16 may be responsible for determining that protocol deviation rates at investigation sites have been within acceptable parameters for past trials. In this example, IRB 16 should not allow the trial to be executed by investigation sites that have protocol deviation rates that are not within acceptable parameters. Accordingly, sponsor 12 may identify, as a failure mode associated with IRB 16 that IRB 16 fails to ensure that protocol deviation rates for investigation sites are within acceptable parameters. In addition, IRB 16 should not allow the trial to continue to be executed at particular investigation sites if the particular investigation sites have protocol deviation rates that are outside acceptable parameters. Accordingly, sponsor 12 may identify, as a failure mode associated with IRB 16 that IRB 16 fails to ensure that protocol deviation rates for investigation sites are within acceptable parameters during the current trial.

In another example, sponsor 12 may generate multiple versions of the trial protocol. In this example, IRB 16 may not correctly handle the different versions. For instance, IRB 16 may approve a non-final version of the trial protocol. Hence, sponsor 12 may identify, as a failure mode associated with IRB 16 that IRB 16 fails to maintain appropriate version control for the trial protocol. Furthermore, some failure modes associated with IRB 16 may involve amendments to the trial protocol. For example, sponsor 12 may identify, as a failure mode associated with IRB 16, that IRB 16 approves the trial on the basis of a wrong version of the trial protocol.

Other failure modes associated with IRB 16 may involve the credentialing of investigator 14. Example credentials include curriculum vitae, licensures, training, etc. For example, sponsor 12 may select investigators that do not have the appropriate credentials for performing the trial. In this example, IRB 16 may be responsible for verifying that the selected investigators have the appropriate credentials. Accordingly, sponsor 12 may identify, as a failure mode associated with IRB 16, that IRB 16 approves the trial when the selected investigators do not have the appropriate credentials. In another example, IRB 16 may be required to have particular credentials in order to review the trial. Accordingly, sponsor 12 may identify, as a failure mode associated with IRB 16, that IRB 16 may approve the trial when IRB 16 does not have the required credentials. Thus, the failure modes identified as being potentially associated with IRB 16 may include approval of the trial by IRB 16 despite IRB 16 or an investigator that performs the trial having inadequate credentials.

Furthermore, other failure modes associated with IRB 16 may involve reports generated by investigator 14 and/or IRB 16. For example, investigator 14 may be responsible for providing reports (e.g., progress report to the IRB after the first five subjects have been enrolled) to IRB 16. In this example, sponsor 12 may identify, as a failure mode associated with IRB 16, that IRB 16 approves continued performance of the trial by investigator 14 despite information in the reports indicating the trial should be discontinued or despite investigator 14 failing to provide such reports. In another example, sponsor 12 may identify, as a failure mode associated with IRB 16, that IRB 16 approves continued performance of the trial by investigator 14 despite the reports from investigator 14 being late beyond acceptable time limits. Thus, the failure modes identified as being potentially associated with IRB 16 may include continued approval of the trial by IRB 16 despite problems with reports provided to IRB 16 by an investigator that performs the trial.

In another example, investigator 14 may be responsible for providing reports to IRB 16 of any deaths, adverse effects, or severe adverse effects. In this example, investigator 14 may fail to provide such reports to IRB 16 or may fail to provide such reports to IRB 16 in a timely manner. In this example, sponsor 12 may identify, as a failure mode associated with IRB 16, that IRB 16 approves continued performing of the trial by investigator 14 despite the investigator's failure to provide such reports or to failure to provide such reports in a timely manner. Furthermore, when IRB 16 receives such a report from investigator 14, IRB 16 may be responsible for providing a response to investigator 14. However, IRB 16 may fail to do so, or may fail to do so in a timely manner. Accordingly, sponsor 12 may identify, as a failure mode associated with IRB 16, that IRB 16 fails to respond to a report of a death, adverse event, or severe adverse event, or fails to respond to such a report in a timely manner.

Other failure modes associated with IRB 16 may involve safety and protocol compliance. For example, IRB 16 may be responsible for determining that investigation sites conform to particular site compliance metrics. Accordingly, sponsor 12 may identify, as a failure mode associated with IRB 16 that IRB 16 fails to ensure that the investigation sites conform to the particular site compliance metrics.

Sponsor 12 may define different detection plans for different types of failure modes. For example, sponsor 12 may define a detection plan for a given failure mode in which occurrences of the given failure mode are to be detected based on information collected from IRB 16. The particular interval may be based on various factors, such as the severity of the risk, ease of detection, likelihood of occurrence, and so on. In another example, sponsor 12 may define a detection plan for a given failure mode in which occurrences of the given failure mode are to be detected by computer system 10 based on an analysis of IRB activity data generated in part by sponsor 12 (or another party) based on data provided by IRB 16. In this example, the detection plan may specify an alert condition associated with the given failure mode. The occurrence of the alert condition may be indicative of the occurrence of the given failure mode. For example, sponsor 12 may identify failure of IRB 16 to verify that investigator 14 has proper credentials as a failure mode associated with IRB 16.

As mentioned above, sponsor 12 may define mitigation procedures for each of the failure modes. The mitigation procedures may include instructions that mitigation teams are to follow when particular failure modes occur. For example, the mitigation procedure may instruct a mitigation term to inform IRB 16 that IRB 16 failed to verify that investigator 14 has proper credentials, and to request IRB 16 to verify the credentials of investigator 14. In another example, a mitigation procedure may indicate that sponsor 12 is to retrain an investigator that performs the trial. In another example, a mitigation procedure may involve starting a trial approval process. In another example, a mitigation procedure may include auditing the investigator that performs the trial. In another example, a mitigation procedure may include suspending enrollment at an investigation site and/or closing the trial at the investigation site. In another example, a mitigation procedure may indicate that IRB 16 is to contact IRB 16 (either directly or through investigator 14) regarding the occurrence of a failure mode. In another example, a mitigation procedure may involve initiating an audit of IRB 16. Furthermore, in some examples, a mitigation procedure may involve obtaining new declarations of consent from trial participants (e.g., to the extent the previous consents may be considered invalid in light of failure mode). In some examples, a mitigation procedure may involve requiring investigator 14 and/or IRB 16 to provide additional verification of the trial-related information (e.g., trial protocol, ICFs, credentials, and/or other documents). Furthermore, in some examples, a mitigation procedure may involve restarting the trial. In another example, a mitigation procedure may involve updating procedures used by investigator 14 and/or IRB 16.

The mitigation procedures may be tailored to the severity of the associated failure modes. For example, a mitigation procedure associated with a study subject safety risk may instruct a monitor (or another team associated with sponsor 12) to conduct an immediate intervention. In some examples, the mitigation procedures may be based on a complexity of the trial protocol, the disease or medical therapy under study, the criticality of the trial population, experience of investigator 14, type and classification of the medical therapy (i.e., investigational product), potential risks, and results of an impact analysis of the trial.

After defining the detection procedures and mitigation procedures for the failure modes associated with IRB 16, sponsor 12 may configure computer system 10 to analyze IRB activity data to detect the alert conditions associated with at least some of the failure modes. In the example of FIG. 1, arrow 32 indicates sponsor 12 providing configuration information to computer system 10. If computer system 10 determines, based on the IRB activity data, that an alert condition has occurred, computer system 10 may output an alert associated with one of the pre-defined mitigation plans. In the example of FIG. 1, arrow 34 indicates computer system 10 outputting an alert to sponsor 12. In some examples, the alert may identify a failure mode, a severity of the failure mode, and a mitigation plan defined for the failure mode. In response to receiving the alert, a mitigation team may perform the mitigation procedures defined for the failure mode associated with the alert.

In this way, a computer system (e.g., computer system 10) may receive, before a start of a trial of a medical therapy, configuration data that configure the computer system to detect alert conditions indicative of occurrences of failure modes identified as being potentially associated with an IRB that reviews the trial. Furthermore, the computer system may receive IRB activity data indicative of activities performed by the IRB. The computer system may detect, based on the IRB activity data that one of the alert conditions has occurred. The alert condition may be indicative of the occurrence of a given one of the failure modes. The computer system may output, in response to detecting that the alert condition has occurred, an alert associated with a mitigation procedure defined for the given failure mode before the start of the trial. The mitigation procedure may indicate a procedure to mitigate the given risk. For instance, the mitigation procedure may indicate that a mitigation team (e.g., a team from sponsor 12) is to intervene in the trial to mitigate the given risk.

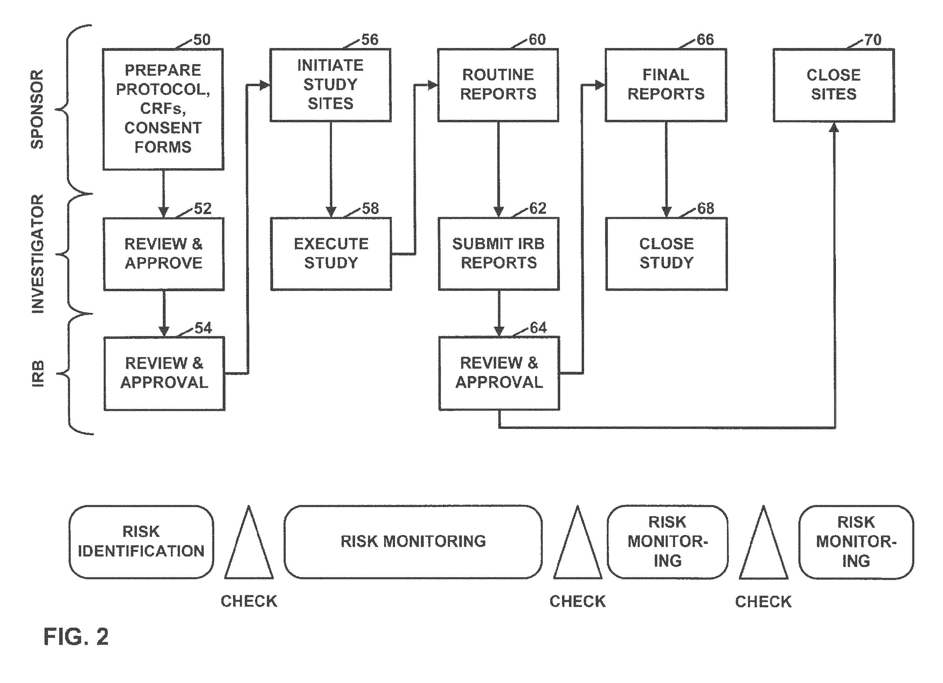

FIG. 2 is a conceptual diagram illustrating an example IRB process, in accordance with one or more techniques of this disclosure. As illustrated in the example of FIG. 2, sponsor 12 prepares a trial protocol for a trial of a medical therapy (50). The trial protocol describes how to conduct the trial. The protocol for the trial may include case report forms (CRFs), informed consent forms, and other types of forms.

After sponsor 12 prepares the trial protocol and it is approved by a regulatory body, investigator 14 may review and approve the trial protocol (52). Following approval of the trial protocol by investigator 14, IRB 16 may review and approve the trial protocol (54).

Following approval of the trial protocol by IRB 16, sponsor 12 may initiate study sites associated with investigator 14 (56). After sponsor 12 initiates the study sites associated with investigator 14, investigator 14 may execute the trial at the study sites (58). As investigator 14 executes the trial, investigator 14 provides routine reports about the trial to sponsor 12 (60). In addition, as investigator 14 executes the trial, investigator 14 submits IRB reports to IRB 16 (62). In different examples, the IRB reports include different content. For instance, the IRB reports may include reports regarding the progress of the trial. In other instances, the IRB reports may include reports of deaths, adverse events, and/or severe adverse events. IRB 16 reviews and, if appropriate, approves the IRB reports submitted by investigator 14 (64).

At the conclusion of the trial, IRB 16 provides data to sponsor 12. The data may include approval by IRB 16 to start or continue the trial. Sponsor 12 may then generate, based in part on the data provided by IRB 16, final reports regarding the trial (66). The final reports regarding the trial may include a summary of events during the trial and results of the trial. In some instances, the reports may include an application to the U.S. F.D.A. (or other authority) based on the trial. In addition, after sponsor 12 generates the final reports, investigator 14 may close the study (68). In addition, IRB 16 may give permission to sponsor 12 to close the investigation sites associated with the trial (70).

As shown in the example of FIG. 2, sponsor 12 may perform risk identification prior to approval of the trial protocol by IRB 16. As part of performing the risk identification, sponsor 12 may identify one or more failure modes and alert conditions associated with the failure modes. Furthermore, in the example of FIG. 2, computer system 10 performs a check after IRB 16 approves the trial protocol. When computer system 10 performs the check, computer system 10 may determine, based at least in part on IRB action data provided by IRB 16, whether alert conditions associated with any of the identified failure modes have occurred. If an alert condition associated with one of the identified failure modes has occurred, sponsor 12 may intervene to mitigate or resolve the failure mode prior to initiating the study sites associated with the trial.

In addition, as shown in the example of FIG. 2, sponsor 12 may perform risk monitoring during the time that investigator 14 is executing the trial. Furthermore, in the example of FIG. 2, when IRB 16 reviews and approves IRB reports submitted to IRB 16 by investigator 14, IRB 16 may provide IRB activity data to computer system 10. Computer system 10 may then determine, based on the provided IRB activity data, whether any of the alert conditions associated with the identified failure modes have occurred. If an alert condition associated with one of the identified failure modes has occurred, sponsor 12 may intervene to mitigate or resolve the failure mode prior to conclusion of the trial.

Sponsor 12 may continue performing risk monitoring until sponsor 12 generates the final reports for the trial. In the example of FIG. 2, computer system 10 may determine, based on the IRB activity data provided over the course of the trial, whether the alert conditions associated with any of the failure modes have occurred. If an alert condition associated with one of the identified failure modes has occurred, sponsor 12 may mitigate or resolve the failure mode prior to submitting the final reports to a regulatory agency, such as the United States F.D.A.

In this way, computer system 10 may receive, after the start of the trial, additional data that reconfigure computer system 10 to detect additional alert conditions. The additional alert conditions may be indicative of occurrences of the failure modes or additional failure modes identified as being potentially associated with the trial. In some instances, computer system 10 may receive the additional data on a periodic basis during the trial.

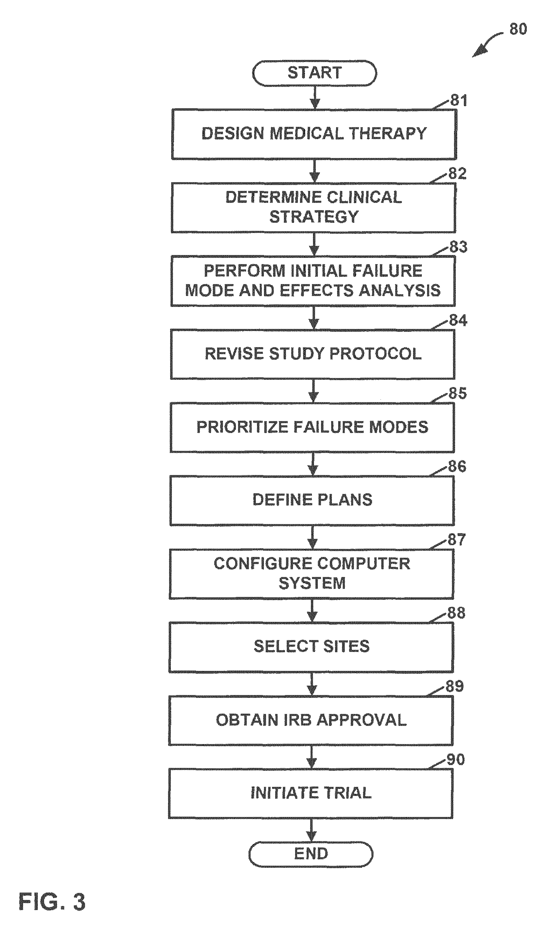

FIG. 3 is a flowchart illustrating an example operation 80 performed by sponsor 12 of a trial of a medical therapy prior to the start of the trial, in accordance with one or more techniques of this disclosure. FIG. 3 is described with respect to the example environment of FIG. 1, however the example of FIG. 3 is not so limited. In other examples, operation 80 may be performed by a CRO delegated by a sponsor or a designated vendor with information technology (IT)/data management capabilities meeting sponsor need. Nevertheless, for ease of explanation, this disclosure assumes that sponsor 12 performs operation 80.

As part of operation 80, sponsor 12 designs a medical therapy (81). As part of designing the medical therapy, sponsor 12 may conduct various types of research regarding the medical therapy, select and analyze components of the medical therapy, conduct bench trials of the medical therapy, and so on.

In addition, sponsor 12 may determine a clinical strategy (82). As part of determining the clinical strategy, sponsor 12 may develop a trial protocol, select and engage investigators (e.g., investigator 14), and perform other tasks associated with designing and preparing to implement a trial of the medical therapy. In some examples, sponsor 12 may start determining the clinical strategy after sponsor 12 has completed designing the medical therapy.

After sponsor 12 determines the clinical strategy, sponsor 12 may perform an initial failure mode and effects analysis (FMEA) (83). During the initial FMEA, sponsor 12 may identify failure modes associated with an IRB (e.g., IRB 16) that is to review the trial. One goal in identifying the failure modes associated with IRB 16 is to narrow the field of potential failure modes to those that are representative of IRBs that review trials of medical therapies for a disease or condition under study, a product malfunction or failure, those having the greatest impact on the product being investigated, those most likely to be a target for a regulatory body audit (e.g., competent authority, U.S. F.D.A. Bioresearch Monitoring (BIMO) inspection or audit), and so on. A BIMO program may be a U.S. F.D.A. program of on-site inspections to ensure the quality and integrity of the data submitted to the U.S. F.D.A. from regulated clinical trials of investigational drugs, devices, and biologics. Inspections may be made both in the U.S. and internationally at clinical investigator sites, IRBs, and sponsors (e.g., pharmaceutical, medical device, and biologic companies, including monitors and CROs working under sponsor 12). As examples, inspections may be conducted by the following F.D.A. Centers under the BIMO program: CDRH - F.D.A. Center for Devices and Radiologic Health, CDER - F.D.A.'s Center for Drug Evaluation and Research, CBER-Center for Biologics Evaluation and Research, and CVM- F.D.A.'s Center for Veterinary Medicine.

In some examples, multiple groups within sponsor 12 may identify the failure modes associated with IRB 16. For instance, one or more business units, study teams, quality assurance teams, data management teams, safety assurance teams, and monitoring teams within sponsor 12 may identify the failure modes associated with IRB 16.

After performing the initial failure mode and effects analysis, sponsor 12 may revise the trial protocol based on the identified failure modes (84). In some instances, sponsor 12 may revise the trial protocol and identify failure modes multiple times.

Next, sponsor 12 may prioritize the failure modes associated with the IRB (85). For instance, sponsor 12 may assign priorities (e.g., low, medium, or high) priorities to the failure modes. In some examples, all failure modes associated with IRB 16 may be classified as severe because they may jeopardize the safety of subjects or the usability of data generated by the trial.

In addition, sponsor 12 may use the identified failure modes to define one or more plans to implement various aspects of the trial (86). Sponsor 12 may identify various detection procedures and mitigation procedures for different failure modes. The detection procedures for other failure modes may involve detection of particular events. The mitigation procedures for the failure modes may be triggered when the particular events occur. For example, a mitigation procedure may be triggered when computer system 10 analyzes meeting minutes of IRB 16 and determines that a majority of IRB members were not present at a meeting of IRB 16 at which IRB 16 approved a trial protocol.

As part of identifying the failure modes associated with IRB 16, sponsor 12 may generate one or more risk assessment documents. In some examples, the risk assessment documents may be of the type described by International Organization for Standardization (ISO) standards no. 14971 and/or 14155. The risk assessment documents may identify some or all areas where there is potential for risk due to IRB failures.

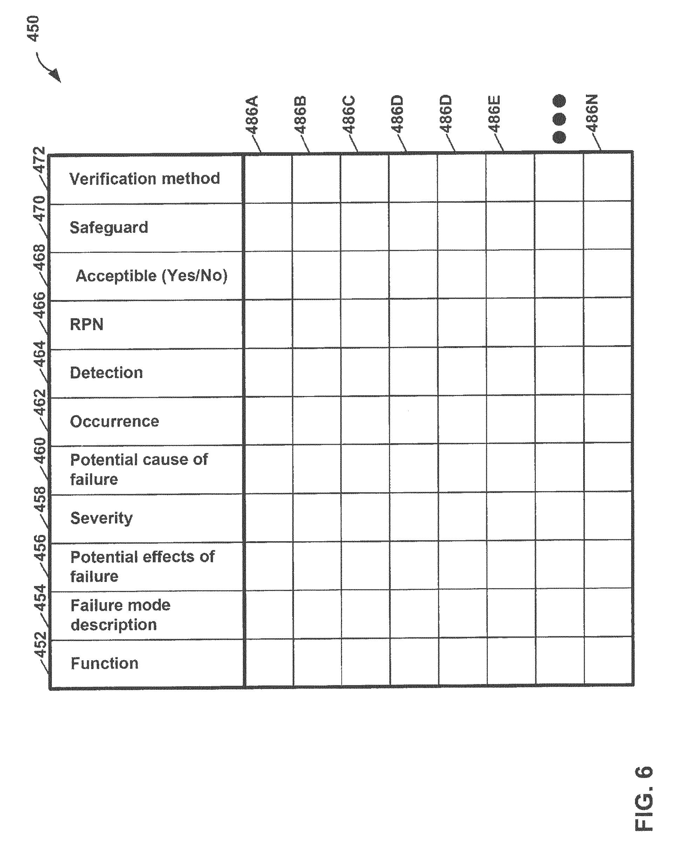

In some examples, a risk assessment document may include a table. Each row of the table may correspond to a different failure mode. The columns of the table may include a function column, a risk description column, an effects column, a severity column, a cause column, an occurrence column, a detection column, a risk priority number (RPN) column, an acceptability column, a safeguard column, and a verification method column. Cells in the function column may indicate functions of the medical therapy or the trial affected by a failure mode. Cells in the failure mode description column may indicate failure mode descriptions that describe the failure modes. Cells in the effects column may indicate effects of the failure modes occurring. Cells in the severity column may indicate severity scores for the failure modes. Failure modes with higher severity scores are anticipated to have more severe effects than failure modes with lower severity scores. Cells in the cause column may indicate potential causes of the failure modes. Cells in the occurrence column may indicate occurrence scores for the failure modes. Failure modes with higher occurrence scores are anticipated to be more likely to occur than failure modes with lower occurrence scores. Cells in the detection column may indicate detection scores for the failure modes. Failure modes with higher detection scores are anticipated to be less detectable than failure modes with low detection scores. Cells in the RPN column may indicate RPNs for the failure modes. The RPN of a failure mode may be equal to the value that results from multiplying the severity, occurrence, and detection scores of the failure mode. Cells in the acceptability column of the table may indicate whether occurrence of the failure mode is or is not acceptable. Cells in the safeguard column may indicate safeguards designed to prevent the occurrence of the failure modes. Cells in the verification method column of the table may indicate methods for verifying whether the failure modes have occurred. FIG. 6, described in detail below, illustrates an example table for identifying failure modes.

Furthermore, sponsor 12 may configure computer system 10 to detect alert conditions and to output alerts (87). Because the alert conditions are associated with identified failure modes, the alerts may also be associated with specified identified failure modes. Sponsor 12 may configure computer system 10 in various ways. For example, sponsor 12 may develop data validation programs that, when executed by the computer system, cause the computer system to identify occurrences of alert conditions for failure modes associated with IRB 16.

In another example, a statistical analysis software package may be installed on computer system 10. In this example, sponsor 12 may develop computer programs (e.g., scripts) that can be run using the statistical analysis software package. Such computer programs may cause the statistical analysis software package to perform statistical analyses to identify patterns or trends within the IRB activity data. Particular patterns, statistical inference or trends within the IRB activity data may be associated with alert conditions for the identified failure modes.

Furthermore, sponsor 12 may perform site selection and engage investigators (88). In addition, sponsor 12 may obtain IRB approval to conduct the trial (89). In some instances, sponsor 12 may obtain IRB approval based on a description of the trial, including a description of the risk and rationales for taking the risks.

After sponsor 12 configures computer system 10, selects investigators, and obtains IRB approval, sponsor 12 may initiate the trial (90). For instance, sponsor 12 may instruct investigators who have met regulatory requirements and received IRB approval (e.g., investigator 14) to begin the trial. Thus, after sponsor 12 has identified the failure modes associated with IRB 16, identified the types and potential severities of the failure modes, determined how occurrences of the failure mode will be minimized, determined how the failure modes are justified in relation to the expected benefits, and after IRB approvals are obtained, sponsor 12 may commence execution of the trial.

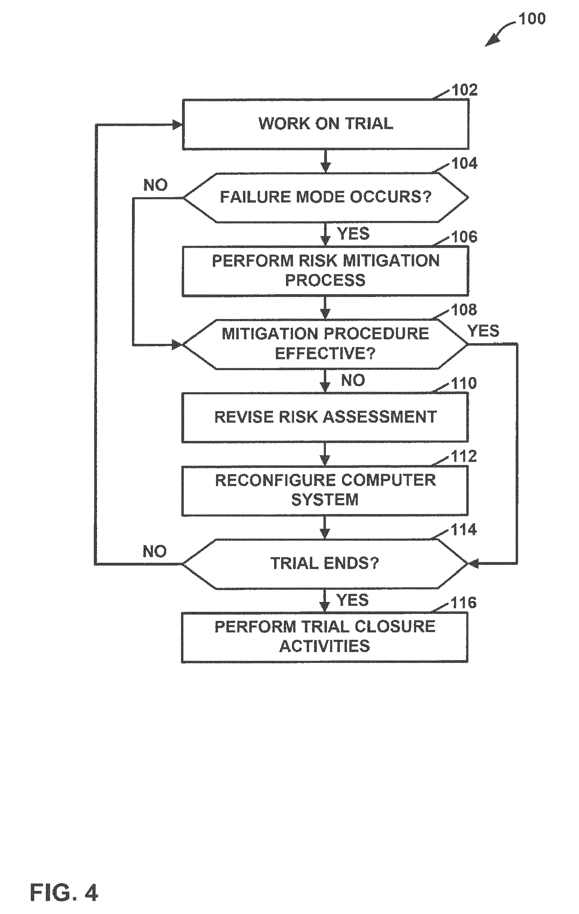

FIG. 4 is a flowchart illustrating an example operation 100 performed in part by a computer system after a trial starts, in accordance with one or more techniques of this disclosure. FIG. 4 is described with respect to the example environment of FIG. 1, however the example of FIG. 4 is not so limited.

After operation 100 begins, one or more investigators (e.g., investigator 14) work on the trial (102). During the trial, it is determined whether a failure mode has occurred (104). The occurrences of various types of failure modes can be determined in various ways. For example, a computer system (e.g., computer system 10) may analyze IRB activity data and detect, based on the IRB activity data that an alert condition associated with a failure mode has occurred. The alert condition may be indicative of the occurrence of the failure mode. In another example, sponsor 12 may determine that the failure mode has occurred.

In response to determining that the failure mode has occurred ("YES" of 104), a mitigation procedure defined for the failure mode can be performed (106). Various parties may perform the mitigation procedure defined for the failure mode. For example, one or more teams within sponsor 12 may perform some or all of the mitigation procedure. After the mitigation procedure has been performed, a determination is made whether the mitigation procedure effectively resolved the occurrence of the failure mode (108). In other words, it may be determined whether the mitigation procedure resolved the failure mode and was sufficient to reduce the probability of the failure mode recurring in the future.

Various parties may determine whether the mitigation procedure was effective. For example, if the failure mode relates to a data management issue, a data management team at the sponsor or sponsor designee may determine whether the mitigation procedure was effective. In another example, if the failure mode relates to a safety issue, a safety team at the sponsor may determine whether the mitigation procedure was effective.

If it is determined that the mitigation procedure was not effective, a risk assessment for the failure mode may be revised (110). Revising the risk assessment for the failure mode may involve revising the mitigation procedure for the failure mode, revising a detection procedure for the failure mode, revising a severity score for the failure mode, revising a priority classification for the failure mode, revising an occurrence score for the failure mode, revising a detectability score for the failure mode, or revising other aspects of the risk assessment for the failure mode. Revising the detection procedure for the failure mode may involve revising an alert condition for the failure mode.

Various parties may revise the risk assessment for the failure mode. For example, if the failure mode relates to a data management issue, a data management team at sponsor 12 may revise the risk assessment. In another example, if the failure mode relates to a regulatory compliance issue, a regulatory compliance team at sponsor 12 may revise the risk assessment.

After revising the risk assessment in step 110, computer system 10 may be reconfigured based on the revised risk assessment (112). For example, a computer program executed by computer system 10 to detect the occurrence of the failure mode may be rewritten based on a revised detection plan in the revised risk assessment for the failure mode.

Various groups may reconfigure computer system 10 based on the revised risk assessment. For example, sponsor 12 may reconfigure computer system 10 based on the revised risk assessment. In another example, sponsor 12 may delegate the task of reconfiguring computer system 10 to one or more other parties, such as a data management vendor or an IT service organization.

After reconfiguring computer system 10 or after determining that the mitigation procedure was effective ("YES" of 108), it is determined whether the trial has ended (114). If the trial has ended ("YES" of 108), trial closure activities may be performed (116). The trial closure activities may include reviewing results, evaluating performance of investigators, preparing reports, submitting applications, and so on.

Various groups may perform the trial closure activities. For example, sponsor 12 may perform some or all of the trial closure activities. The trial closure activities may include preparing reports regarding the trial, closing investigation sites, analyzing trial methodology, evaluating effectiveness of investigation sites, and so on. In another example, sponsor 12 may delegate one or more of the trial closure activities to one or more other parties, such as monitoring organizations, independent contractors or auditors.

On the other hand, if the trial has not yet ended ("NO" of 114), investigator 14 may continue work on the trial (102). During the trial, it may be determined whether mitigation procedures are effective (108), even if no failure mode occurs. For instance, if no failure mode occurs ("NO" of 104), sponsor 12 may still determine whether the mitigation procedures are effective (108). For example, sponsor 12 may periodically determine, during the trial, whether mitigation procedures for various failure modes are effective.

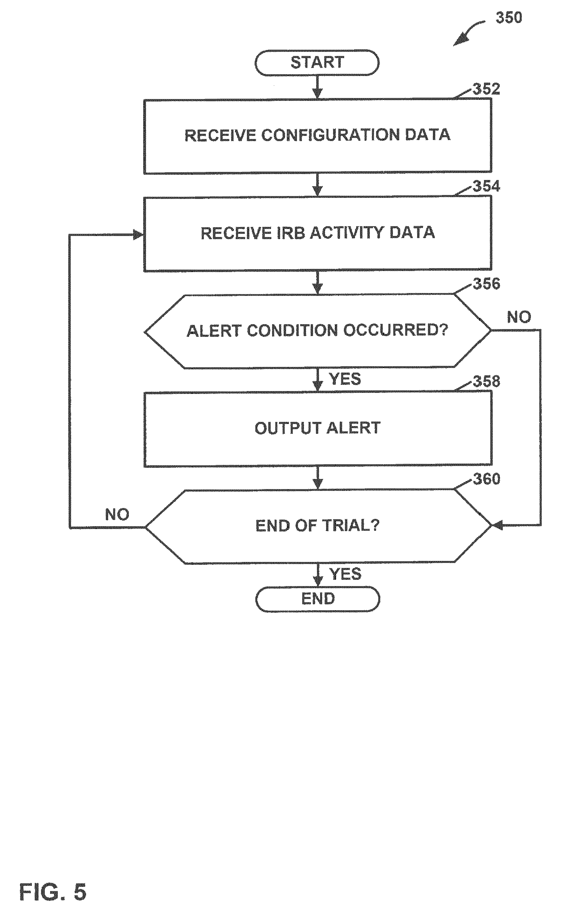

FIG. 5 is a flowchart illustrating an example operation 350 performed by a computer system, in accordance with one or more techniques of this disclosure. FIG. 5 is described with respect to the example environment of FIG. 1, however the example of FIG. 5 is not so limited. In some examples, computer system 10 of FIG. 1 may perform operation 350.

In the example of FIG. 5, computer system 10 receives configuration data before a start of a trial of a medical therapy (352). The configuration data may configure computer system 10 to detect alert conditions indicative of occurrences of risks associated with an IRB (e.g., IRB 16) that reviews the trial. Computer system 10 may receive the configuration data from various sources. For example, computer system 10 may receive the configuration data via a communication network from one or more computers used by sponsor 12. In another example, the configuration data may be generated by one or more entities, such as sponsor 12, and stored on one or more physical computer-readable storage media. In this example, computer system 10 may read the configuration data from the physical computer-readable storage media. In another example, computer system 10 may receive user input to configure computer system 10 to detect the alert conditions.

The configuration data may configure computer system 10 in various ways. For example, the configuration data may comprise a set of rules. Each of the rules may specify a condition and an action to be performed when the condition is satisfied. In some instances, the configuration data may include instructions that indicate to computer system 10 how to determine whether the conditions of the rules are satisfied.

Furthermore, the configuration data may include one or more computer programs or scripts. Such computer program or scripts may include software instructions that, when executed, configure computer system 10 to detect the alert conditions associated with various failure modes and may configure computer system 10 to output alerts associated with the failure modes.

Subsequently, computer system 10 may receive IRB activity data generated during conduct of the trial (354). In some examples, the IRB activity data may be generated by IRB 16 prior to approval of the trial and/or during conduct of the trial. Thus, computer system 10 may receive an initial portion of the IRB activity data following an initial approval of the trial by IRB 16. In addition, computer system 10 may receive one or more additional portions of the IRB activity data during execution of the trial. Computer system 10 may receive the IRB activity data in various ways. For example, computer system 10 may receive the IRB activity data through a system of web-based forms displayed by web browser applications executed on computers used by IRB 16. In another example, computer system 10 may receive the IRB activity data through special-purpose applications. In some examples, computer system 10 may receive electronic report forms that contain the IRB activity data. When computer system 10 receives the IRB activity data, computer system 10 may store the IRB activity data in a database.

Furthermore, after the trial begins, computer system 10 may determine, based on the IRB activity data, whether any of the alert conditions have occurred (356). For example, computer system 10 may detect that a particular alert condition has occurred when the IRB activity data indicates that a majority of IRB members were not present during meetings of the IRB.

In some examples, computer system 10 may determine, on a real-time basis, whether the alert conditions have occurred. For instance, computer system 10 may determine whether the alert conditions have occurred in response to receiving the IRB activity data. In other examples, computer system 10 may determine in batch mode whether the alert conditions have occurred.

Computer system 10 may determine in various ways whether any of the alert conditions have occurred. For example, each of the alert conditions may be expressed as one or more condition-action rules. In this example, computer system 10 may determine that an alert condition has occurred when the conditions of the rules are satisfied.

In response to detecting that an alert condition has occurred ("YES" of 356), computer system 10 may output an alert associated with the alert condition (358). The alert may be associated with a pre-defined mitigation procedure. In some examples, the alert may indicate or otherwise identify the mitigation procedure. For instance, in such examples, the alert may indicate that the mitigation procedure instructs a team from sponsor 12 to retrain investigator 14. In other examples, the alert may include information, such as a failure mode identifier, that enables a mitigation team to determine the pre-defined mitigation procedure.

Computer system 10 may output the alert in various ways. For example, computer system 10 may output the alert by sending an email, fax, instant message, text message, or other type of written alert to a team associated with sponsor 12. In another example, computer system 10 may output the alert by including or enabling the alert to be included in a webpage. In another example, computer system 10 may display the alert along with (e.g., next to) a questionable element of the IRB activity data.

Computer system 10 may output the alert to various groups, depending on the mitigation plan defined for the failure mode associated with the alert condition. For example, if the mitigation procedure is to be performed at least in part by a monitoring organization that monitors the conduct of the trial, computer system 10 may output the alert to the monitoring organization. In this example, the mitigation procedure may require a site visit to be conducted. In another example, if the mitigation procedure is to be performed at least in part by sponsor 12, computer system 10 may output the alert to sponsor 12.

In other examples, computer system 10 may output an indication of the mitigation procedure separately from the alert. For example, computer system 10 may output an indication of the mitigation procedure in response to a request or command received by computer system 10. In this example, computer system 10 may receive such a request or command in various ways. For instance, computer system 10 may receive such a request or command via a web-based interface, a command-line interface, or another type of interface. The indication of the mitigation procedure may include text that describes some or all aspects of the mitigation procedure. In other examples, the indication of the mitigation procedure may include data that identifies printed material describing some or all aspects of the mitigation procedure.

After outputting the alert in step 358 or after determining that no alert condition has occurred ("NO" of 356), computer system 10 may determine whether the trial has ended (360). If the trial has not yet ended, computer system 10 may receive additional IRB activity data (354) and steps 356, 358, and 360 may recur.

FIG. 6 is a conceptual diagram illustrating an example table 450 for identifying failure modes. A sponsor of a trial of a medical therapy (e.g., sponsor 12) may use table 450 to identify failure modes associated with an IRB prior to the start of the trial. In the example of FIG. 6, table 450 has eleven columns: a function column 452, a failure mode description column 454, a potential effects of failure column 456, a severity column 458, a potential cause of failure column 460, an occurrence column 462, a detection column 464, a risk priority number (RPN) column 466, an acceptability column 468, a safeguard column 470, and a verification column 472. Table 450 has a plurality of rows 486A-486N (collectively, "rows 486"). Each of rows 486 may correspond to a different failure mode identified by sponsor 12.

Sponsor 12 may insert in function column 452 descriptions of study function impacted by identified failure modes. For instance, if the failure mode relates to a pattern of protocol violations by investigator 14 , sponsor 12 may insert the label "protocol violations" in the appropriate cell of function column 452. If the failure mode relates to a failure to provide particular elements, sponsor 12 may insert the label "required elements" in the appropriate cell of function column 452.

Furthermore, sponsor 12 may insert descriptions of the identified failure modes in appropriate cells of column 454. For example, if a failure mode relates to a failure of an investigator to provide particular elements, sponsor 12 may describe these elements in the appropriate cell of failure mode column 454. Sponsor 12 may insert descriptions of effects of the identified failure mode on patients and/or user in column 456. In addition, sponsor 12 may insert severity scores for the identified failure modes in appropriate cells of severity column 458. Failure modes with higher severity scores are anticipated to have more severe effects than failure modes with lower severity scores.

Sponsor 12 may also identify one or more potential causes of the identified failure modes. Sponsor 12 may then insert descriptions of the identified potential causes of the identified failure modes in appropriate cells of column 460. In addition, sponsor 12 may assign occurrence scores to the identified failure modes and insert the occurrence scores into appropriate cells of occurrence score column 462. Sponsor 12 may assign higher occurrence scores to failure modes with higher probabilities of occurring.

In addition, sponsor 12 may also assign detection scores to the identified failure modes and insert the detection scores into appropriate cells of detection score column 464. Sponsor 12 may assign higher detection scores to failure modes that are more difficult to detect than to failure modes that are relatively less difficult to detect. Sponsor 12 may assign RPNs to the identified failure modes and may insert the RPNs into appropriate cells of column 466. The RPN of the failure mode may be equal to the value that results from multiplying the severity, occurrence, and detection scores of the failure mode. Furthermore, sponsor 12 may determine whether occurrences of the identified failure modes are acceptable. Sponsor 12 may insert indications (e.g., yes or no) appropriate cells in column 468 to indicate whether occurrences of the failure modes are acceptable. Furthermore, before the start of the trial, sponsor 12 may identify safeguards for identified failure modes. Sponsor 12 may then insert descriptions of the safeguards into appropriate cells of column 474.

In one example, rows 486A and 486B of table 450 may relate to failure modes associated with ICFs. In this example, sponsor 12 may enter the following data in row 486A: "required elements" in function column 452; "missing required elements at initial informed consent form (ICF) submission to IRB" in failure mode description column 454; "patient not informed of study risks/benefits" in potential effects of failure column 456; 1, 3, or 9 in severity column 458; "sponsor did not confirm the required elements of consent" in potential cause of failure column 460; 1, 3, or 9 in occurrence column 462; 1, 3, or 9 in detection column 464; an RPN in RPN column 466; "No" in acceptability column 468; "IRB review" in safeguard column 470; and "IRB reported rate of failure mode to sponsor" in verification method column 472.

Furthermore, in this example, sponsor 12 may enter the following data in row 486B: "required elements" in function column 452; "IRB amends ICF and omits required elements" in failure mode description column 454; "patient not informed of study risks/benefits" in potential effects of failure column 456; 1, 3, or 9 in severity column 458; "IRB did not confirm elements of consent" in potential cause of failure column 460; 1, 3, or 9 in occurrence column 462; 1, 3, or 9 in detection column 464; an RPN in RPN column 466; "No" in acceptability column 468; "Sponsor verification of IRB modified consents" in safeguard column 470; and "Sponsor reported rate of IRB failure mode" in verification method column 472.

Furthermore, in this example, rows 486C-486E may relate to the trial protocol. In this example, sponsor 12 may enter the following data in row 486C: "required elements" in function column 452; "missing required elements at initial protocol submission to IRB" in failure mode description column 454; "study design/integrity risk" in potential effects of failure column 456; 1, 3, or 9 in severity column 458; "Sponsor did not confirm protocol met design or integrity criteria" in potential cause of failure column 460; 1, 3, or 9 in occurrence column 462; 1, 3, or 9 in detection column 464; an RPN in RPN column 466; "No" in acceptability column 468; "IRB review" in safeguard column 470; and "IRB reported rate of failure mode to sponsor" in verification method column 472.

Furthermore, in this example, sponsor 12 may enter the following data in row 486D "protocol violations" in function column 452; "Clinical site has recurring rate of protocol violations" in failure mode description column 454; "study integrity and patient safety risk" in potential effects of failure column 456; 1, 3, or 9 in severity column 458; "investigator non-compliance" in potential cause of failure column 460; 1, 3, or 9 in occurrence column 462; 1, 3, or 9 in detection column 464; an RPN in RPN column 466; "No" in acceptability column 468; "IRB review" in safeguard column 470; and "IRB shuts down site" in verification method column 472.

Furthermore, in this example, sponsor 12 may enter the following data in row 486E: "protocol violations" in function column 452; "clinical site fails to report violations" in failure mode description column 454; "study integrity and patient safety risk" in potential effects of failure column 456; 1, 3, or 9 in severity column 458; "investigator non-compliance" in potential cause of failure column 460; 1, 3, or 9 in occurrence column 462; 1, 3, or 9 in detection column 464; an RPN in RPN column 466; "No" in acceptability column 468; "IRB review" in safeguard column 470; and "IRB shuts down site" in verification method column 472.

In some examples, a computer system (e.g., computer system 10) may provide a graphical user interface for entering data in table 450. In other examples, sponsor 12 may generate table 450 offline and may then use table 450 as a basis for configuring computer system 10 to detect alert conditions.

FIG. 7 is a block diagram of an example configuration of computer system 10. In the example of FIG. 7, computer system 10 comprises a computing device 500 and one or more other computing devices. Computing device 500 is a physical device that processes information. In the example of FIG. 7, computing device 500 comprises a data storage system 502, a memory 504, a secondary storage system 506, a processing system 508, an input interface 510, a display interface 512, a communication interface 514, and one or more communication media 516. Communication media 516 enable data communication between processing system 508, input interface 510, display interface 512, communication interface 514, memory 504, and secondary storage system 506. Computing device 500 can include components in addition to those shown in the example of FIG. 7. Furthermore, some computing devices do not include all of the components shown in the example of FIG. 7.

A computer-readable medium may be a medium from which a processing system can read data. Computer-readable media may include computer storage media and communications media. Computer storage media may include physical devices that store data for subsequent retrieval. Computer storage media are not transitory. For instance, computer storage media do not exclusively comprise propagated signals. Computer storage media may include volatile storage media and non-volatile storage media. Example types of computer storage media may include random-access memory (RAM) units, read-only memory (ROM) devices, solid state memory devices, optical discs (e.g., compact discs, DVDs, BluRay discs, etc.), magnetic disk drives, electrically-erasable programmable read-only memory (EEPROM), programmable read-only memory (PROM), magnetic tape drives, magnetic disks, and other types of devices that store data for subsequent retrieval. Communication media may include media over which one device can communicate data to another device. Example types of communication media may include communication networks, communications cables, wireless communication links, communication buses, and other media over which one device is able to communicate data to another device.

Data storage system 502 may be a system that stores data for subsequent retrieval. In the example of FIG. 7, data storage system 502 comprises memory 504 and secondary storage system 506. Memory 504 and secondary storage system 506 may store data for later retrieval. In the example of FIG. 7, memory 504 stores computer-executable instructions 518 and program data 520. Secondary storage system 506 stores computer-executable instructions 522 and program data 524. Physically, memory 504 and secondary storage system 506 may each comprise one or more computer storage media.

Processing system 508 is coupled to data storage system 502. Processing system 508 may read computer-executable instructions from data storage system 502 and executes the computer-executable instructions. Execution of the computer-executable instructions by processing system 508 may configure and/or cause computing device 500 to perform the actions indicated by the computer-executable instructions. For example, execution of the computer-executable instructions by processing system 508 can configure and/or cause computing device 500 to provide Basic Input/Output Systems, operating systems, system programs, application programs, or can configure and/or cause computing device 500 to provide other functionality.

Processing system 508 may read the computer-executable instructions from one or more computer-readable media. For example, processing system 508 may read and execute computer-executable instructions 518 and 522 stored on memory 504 and secondary storage system 506.

Processing system 508 may comprise one or more processing units 526. Processing units 526 may comprise physical devices that execute computer-executable instructions. Processing units 526 may comprise various types of physical devices that execute computer-executable instructions. For example, one or more of processing units 526 may comprise a microprocessor, a processing core within a microprocessor, a digital signal processor, a graphics processing unit, or another type of physical device that executes computer-executable instructions.

Input interface 510 may enable computing device 500 to receive input from an input device 528. Input device 528 may comprise a device that receives input from a user. Input device 528 may comprise various types of devices that receive input from users. For example, input device 528 may comprise a keyboard, a touch screen, a mouse, a microphone, a keypad, a joystick, a brain-computer interface device, or another type of device that receives input from a user. In some examples, input device 528 is integrated into a housing of computing device 500. In other examples, input device 528 is outside a housing of computing device 500. In some examples, input device 528 may receive configuration data, IRB activity data, and/or other types of data as described above.

Display interface 512 may enable computing device 500 to display output on a display device 530. Display device 530 may be a device that displays output. Example types of display devices include monitors, touch screens, display screens, televisions, and other types of devices that display output. In some examples, display device 530 is integrated into a housing of computing device 500. In other examples, display device 530 is outside a housing of computing device 500. In some examples, display device 530 may display alerts or other types of data as described above.

Communication interface 514 may enable computing device 500 to send and receive data over one or more communication media. Communication interface 514 may comprise various types of devices. For example, communication interface 514 may comprise a Network Interface Card (NIC), a wireless network adapter, a Universal Serial Bus (USB) port, or another type of device that enables computing device 500 to send and receive data over one or more communication media. In some examples, communication interface 514 may receive configuration data, IRB activity data, and/or other types of data as described above. Furthermore, in some examples, communication interface 514 may output alerts, queries, and/or other types of data as described above.

The techniques described in this disclosure may be implemented, at least in part, in hardware, software, firmware, or any combination thereof. For example, various aspects of the described techniques may be implemented within one or more processors, including one or more microprocessors, digital signal processors (DSPs), application specific integrated circuits (ASICs), field programmable gate arrays (FPGAs), or any other equivalent integrated or discrete logic circuitry, as well as any combinations of such components. The term "processor" or "processing circuitry" may generally refer to any of the foregoing logic circuitry, alone or in combination with other logic circuitry, or any other equivalent circuitry. A control unit including hardware may also perform one or more of the techniques of this disclosure.

Such hardware, software, and firmware may be implemented within the same device or within separate devices to support the various techniques described in this disclosure. In addition, any of the described units, modules or components may be implemented together or separately as discrete but interoperable logic devices. Depiction of different features as modules or units is intended to highlight different functional aspects and does not necessarily imply that such modules or units must be realized by separate hardware, firmware, or software components. Rather, functionality associated with one or more modules or units may be performed by separate hardware, firmware, or software components, or integrated within common or separate hardware, firmware, or software components.

The techniques described in this disclosure may also be embodied or encoded in a computer-readable medium, such as a computer-readable storage medium, containing instructions. Instructions embedded or encoded in a computer-readable medium, including a computer-readable storage medium, may cause one or more programmable processors, or other processors, to implement one or more of the techniques described herein, such as when instructions included or encoded in the computer-readable medium are executed by the one or more processors. Computer readable storage media may include random access memory (RAM), read only memory (ROM), programmable read only memory (PROM), erasable programmable read only memory (EPROM), electronically erasable programmable read only memory (EEPROM), flash memory, a hard disk, a compact disc ROM (CD-ROM), a floppy disk, a cassette, magnetic media, optical media, or other computer readable media. In some examples, an article of manufacture may comprise one or more computer-readable storage media.

Various examples have been described. These and other examples are within the scope of the following claims.

## Claims

1. A method comprising:
receiving, by a computer system (10), before a start of a trial of a medical therapy, data that configure the computer system to detect an alert condition indicative of an occurrence of a failure mode identified as associated with an Institutional Review Board (IRB) (16) review of the trial, wherein a mitigation procedure is defined for the failure mode before the start of the trial, the mitigation procedure indicating a procedure to mitigate the occurrence of the failure mode;
receiving, by the computer system, IRB activity data indicative of activities performed by the IRB;
detecting, by the computer system based on the IRB activity data, that the alert condition has occurred; and
in response to detecting the alert condition has occurred, outputting, by the computer system, an alert.

2. The method of claim 1, wherein receiving the data that configure the computer system to detect the alert condition comprises receiving software instructions that configure the computer system to detect the alert condition.

3. The method of claim 1, wherein receiving the data that configure the computer system to detect the alert condition comprises receiving user input to configure the computer system to detect the alert condition.

4. The method of claim 1, wherein the failure mode includes:
a failure by the IRB to ensure that informed consent forms used in the trial include all required elements;
a failure by the IRB to ensure that informed consent forms conform to local requirements;
approval of the trial by the IRB despite a conflict of interest by the IRB or an investigator that executes the trial;
approval of the trial by the IRB despite missing or incorrect elements in a trial protocol for the trial;
approval of the trial by the IRB despite the IRB or an investigator that performs the trial having inadequate credentials; and/or
continued approval of the trial by the IRB despite problems with reports provided to the IRB by an investigator that performs the trial.

5. The method of any preceding claim, wherein the IRB activity data include one or more of: data indicating meeting minutes of the IRB, credentials of the IRB, and reports generated by the IRB to a sponsor of the trial.

6. The method of any preceding claim, wherein receiving the IRB activity data comprises
receiving, by the computer system, an initial portion of the IRB activity data following an initial approval of the trial by the IRB; and
receiving, by the computer system, one or more additional portions of the IRB activity data during execution of the trial.

7. The method of any preceding claim, wherein the mitigation procedure includes one or more of: restarting the trial approval process, retraining an investigator that performs the trial, auditing the IRB, auditing the investigator, obtaining new declarations of consent from trial participants, requiring the IRB or the investigator to provide additional verification of trial-related information, suspending enrollment at an investigation site, and closing the trial at the investigation site.

8. The method of any preceding claim, wherein the alert comprises a message that identifies the mitigation procedure associated with the failure mode.

9. The method of any preceding claim, wherein generating the alert comprises generating, by the computer system, the alert prior to the start of the trial.

10. The method of any of claims 1 to 8, wherein generating the alert comprises generating, by the computer system, the alert after the start of the trial and prior to an end of the trial.

11. A computer system that comprises one or more processing units that are configured to:
receive, before a start of a trial of a medical therapy, data that configures the computer system to detect an alert condition indicative of an occurrence of a failure mode identified as associated with an Institutional Review Board (IRB) review of the trial, wherein a mitigation procedure is defined for the failure mode before the start of the trial, the mitigation procedure indicating a procedure to mitigate the occurrence of the failure mode;
receive IRB activity data indicative of activities performed by the IRB;
detect, based on the IRB activity data, that the alert condition has occurred; and
in response to detecting the alert condition has occurred, output an alert.

12. The computer system of claim 11, wherein the failure mode includes one or more of:
a failure by the IRB to ensure that informed consent forms conform to local requirements,
approval of the trial by the IRB despite a conflict of interest by the IRB or an investigator that performs the trial,
approval of the trial by the IRB despite missing or incorrect elements in a trial protocol for the trial,
approval of the trial by the IRB despite the IRB or the investigator that performs the trial having inadequate credentials, and
approval of the trial by the IRB despite problems with reports provided to the IRB by the investigator that performs the trial.

13. A non-transitory computer-readable storage medium that stores computer-executable instructions that, when executed, configure a computer system to:
receive, before a start of a trial of a medical therapy, data that configures the computer system to detect an alert condition indicative of an occurrence of a failure mode identified as associated with an Institutional Review Board (IRB) review of the trial, wherein a mitigation procedure is defined for the failure mode before the start of the trial, the mitigation procedure indicating a procedure to mitigate the occurrence of the failure mode;
receive IRB activity data indicative of activities performed by the IRB;
detect, based on the IRB activity data, that the alert condition has occurred; and
in response to detecting that the alert condition has occurred, output an alert.

14. The non-transitory computer-readable storage medium of claim 13, wherein the failure mode includes one or more of:
a failure by the IRB to ensure that informed consent forms conform to local requirements,
approval of the trial by the IRB despite a conflict of interest by the IRB or an investigator that performs the trial,
approval of the trial by the IRB despite missing or incorrect elements in a trial protocol for the trial,
approval of the trial by the IRB despite the IRB or the investigator that performs the trial having inadequate credentials, and
approval of the trial by the IRB despite problems with reports provided to the IRB by the investigator that performs the trial.
